# FASCICULE DE BREVET EUROPEEN

(11) **EP 0 738 701 B1**
(45) Date de publication et mention de la délivrance du brevet: **01.09.1999**
(21) Numéro de dépôt: 96420114.9
(22) Date de dépôt: 04.04.1996
(51) Int. Cl.: C07C 57/03, C07C 51/14

(54) **Procédé d'hydroxycarbonylation du butadiène**
Verfahren zur Carbonylisierung von Butadien
Process for the carbonylation of butadiene

(30) Priorité: 20.04.1995 FR 9504949
(43) Date de publication de la demande: 23.10.1996
(73) Titulaire: RHODIA FIBER & RESIN INTERMEDIATES, 92408 Courbevoie Cédex (FR)
(72) Inventeur: Patois, Carl, 69003 Lyon (FR); Perron, Robert, 69390 Charly (FR)
(74) Mandataire: Vignally, Noel

(56) Documents cités:
- FR-A- 2 158 171
- US-A- 4 000 170

## Description

La présente invention conceme l'hydroxycarbonylation du butadiène, par réaction avec le monoxyde de carbone et l'eau, afin de préparer les acides penténoïques.

Une des voies possibles d'accès à l'acide adipique, qui est l'un des deux constituants de base du polyamide 6-6, réside dans la double carbonylation du butadiène.

Bien qu'il puisse être imaginé de réaliser en une seule étape les deux réactions d'hydroxycarbonylation menant du butadiène à l'acide adipique, il s'avère en pratique que ces deux réactions doivent être conduites au moins en partie successivement, si l'on veut obtenir des sélectivités suffisamment élevées pour permettre d'envisager un procédé industriel économiquement viable.

Le brevet US 3 579 551 a décrit un procédé de transformation d'un composé comportant au moins une double liaison éthylénique en acide carboxylique, par réaction avec le monoxyde de carbone et l'eau, en présence d'un composé de l'iridium et d'un promoteur iodé, à une température de 50°C à 330°C et sous une pression partielle de monoxyde d'environ 0,3 à 210 bars.

Dans le procédé décrit, il semble que n'importe quel composé de l'iridium puisse être utilisé et divers types de composés iodés sont mentionnés ; le rapport molaire iode/iridium peut varier dans de très larges limites, de 1/1 à 2500/1 et de préférence de 3/1 à 300/1.

Le milieu réactionnel peut renfermer tout solvant compatible avec le système catalytique, les acides monocarboxyliques ayant de 2 à 20 atomes de carbone, tels que les acides acétique, propionique, hexanoïque, décanoïque, dodécanoïque, naphtoïque, oléique ou trans-9-octadécénoïque, étant préférés.

Les exemples illustrant ce brevet montrent que le procédé tel défini conduit de manière prépondérante à l'acide carboxylique ramifié : ainsi l'hydroxycarbonylation du propylène donne majoritairement l'acide isobutyrique (exemple 1) tandis que l'hydroxycarbonylation de l'hexène-1 donne majoritairement des acides en C7 ramifiés (exemple 19).

Dans le brevet US 3 816 489, qui a les mêmes inventeurs que le brevet discuté précédemment et qui en découle directement, il a été proposé de mettre en oeuvre un rapport molaire iode/iridium de 3/1 à 100/1 afin d'essayer d'obtenir de manière prédominante l'acide carboxylique linéaire. Appliquée à des mono-oléfines telles que l'hexène-1, le pentène-1, le pentène-2, le dodécène-1, cette technique semble conduire au résultat recherché. Aucun exemple de ce brevet n'est réalisé avec un diène.

Si cependant l'on met en oeuvre le procédé selon le brevet US 3 759 551 pour hydroxycarbonyler un diène tel que le butadiène, on constate que la sélectivité en acides penténoïques, produits visés, est très faible, sinon nulle et que l'on obtient essentiellement les acides saturés correspondants, c'est-à-dire l'acide valérique et l'acide méthylbutyrique.

Le brevet EP-A-0 405 433 porte sur l'hydroxycarbonylation du butadiène en acides penténoïques, en présence d'un catalyseur au rhodium et d'un promoteur bromure ou iodure, dans un solvant acide carboxylique. Ce procédé met en oeuvre un catalyseur au rhodium, métal particulièrement coûteux.

Le problème de l'hydroxycarbonylation du butadiène en acides penténoïques n'est donc pas résolu de manière économiquement satisfaisante par les techniques décrites dans l'art antérieur.

La présente invention propose une solution à ce problème.

Plus précisément, l'invention consiste en un procédé d'hydroxycarbonylation du butadiène, par réaction avec le monoxyde de carbone et l'eau, à une température supérieure à 30°C, sous une pression partielle en monoxyde de carbone mesurée à 25°C égale ou supérieure à 0,5 bar, en présence d'un catalyseur à base d'iridium et d'un promoteur iodé ou bromé, dans un milieu solvant, caractérisé en ce que le rapport molaire iode ou brome/iridium est inférieur ou égal à 20/1 et la concentration pondérale en eau du mélange réactionnel est inférieure ou égale à 8 %.

De préférence, la concentration pondérale en eau dans le mélange réactionnel est comprise entre 0,00001 % et 5 % .

De manière encore préférée, la concentration pondérale en eau dans le mélange réactionnel est comprise entre 0,01 % et 2 %.

Une variante intéressante du procédé de l'invention consiste à injecter l'eau au fur et à mesure de l'avancement de la réaction, ce qui permet de maintenir sa concentration dans le mélange réactionnel à une valeur très faible, tout en permettant à la réaction d'hydroxycarbonylation de s'effectuer.

La limite inférieure de concentration en eau est donc donnée à titre indicatif, car elle peut être très faible à un moment donné, en particulier dans l'hypothèse envisagée précédemment d'une injection continue de l'eau, qui est alors rapidement transformée.

Le substrat mis en oeuvre, le butadiène, peut contenir minoritairement des composés dérivés comme les buténols allyliques, tels que le 3-butène-2-ol, le 2-butène-1-ol et leurs mélanges.

De préférence, le butadiène représente au moins 80 % en poids du mélange butadiène/dérivés du butadiène et encore plus préférentiellement au moins 90 % .

La concentration en butadiène du mélange est aussi un paramètre important à prendre en compte dans la mise en oeuvre du procédé de l'invention. Elle est de préférence maintenue à une valeur inférieure ou égale à 16 % en poids par rapport au poids total du mélange réactionnel.

La concentration en butadiène peut être très faible de manière instantanée, notamment en cas d'injection continue du butadiène qui est alors transformé très rapidement. Il n'y a donc pas de limite inférieure critique pour cette concentration qui dépend notamment du mode d'introduction du butadiène.

De préférence, la concentration en butadiène dans le mélange réactionnnel est inférieure ou égale à 11 % en poids par poids et encore plus préférentiellement inférieure ou égale à 5,5 % en poids par poids.

Pour le catalyseur à l'iridium nécessaire dans le présent procédé, diverses sources d'iridium sont susceptibles d'être mises en oeuvre.

A titre d'exemples de telles sources d'iridium, on peut citer :
- Ir métallique; IrO₂ ; Ir₂O₃ ;
- IrCl₃ ; IrCl₃, 3H₂O ;
- IrBr₃ ; IrBr₃, 3H₂O ;
- les iodures d'iridium ;
- les carboxylates d'iridium, notamment l'acétate d'lr ou le penténoate d'Ir ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂I ;
- Ir(CO)[P(C₆H₅)₃]₂Cl ;
- Ir[P(C₆H₅)₃]₃I ;
- HIr[P(C₆H₅)₃]₃(CO) ;
- Ir(acac)(CO)₂ ;
- [IrCI(cod)]₂ ;
   (acac = acétylacétonate ; cod = cyclooctadiène-1,5).

La quantité de catalyseur à mettre en oeuvre peut varier dans de larges limites.

En général, une quantité, exprimée en moles d'iridium métal par litre de mélange réactionnel, comprise entre 10⁻⁴ et 10⁻¹ conduit à des résultats satisfaisants. Des quantités inférieures peuvent être utilisées, mais on observe toutefois que la vitesse de réaction est faible. Des quantités supérieures n'ont des inconvénients qu'au plan économique.

De préférence, la concentration en iridium dans le mélange réactionnel est comprise entre 5.10⁻⁴ et 5.10⁻² mole/litre.

Par promoteur iodé ou bromé, on entend dans le cadre du procédé l'iodure d'hydrogène, le bromure d'hydrogène et les composés organo-iodés et organo-bromés capables de générer respectivement l'iodure d'hydrogène et le bromure d'hydrogène dans les conditions réactionnelles; ces composés organo-iodés et organo-bromés sont plus particulièrement les iodures et les bromures d'alkyle ayant de 1 à 10 atomes de carbone, parmi lesquels l'iodure de méthyle et le bromure de méthyle sont préférés.

Parmi les promoteurs, on préfère de manière générale les promoteurs iodés.

Le rapport molaire iode ou brome/iridium est de préférence compris entre 1/1 et 10/1.

Plus préférentiellement, le rapport molaire iode ou brome/iridium est compris entre 1/1 et 5/1.

La limite inférieure indiquée pour le rapport molaire iode ou brome/iridium n'a pas de caractère critique pour la réaction. Ce rapport peut être inférieur à 1, mais dans un tel cas les inconvénients sont économiques, car une partie de l'iridium n'est pas activée et n'a guère de rôle catalytique.

Une autre caractéristique du présent procédé réside dans l'utilisation d'un solvant, liquide dans les conditions de la réaction d'hydroxycarbonylation du butadiène.

Ce solvant peut être de nature très variée. On peut choisir notamment les acides carboxyliques, les hydrocarbures aliphatiques, les hydrocarbures cycloaliphatiques, les hydrocarbures aromatiques, les hydrocarbures aliphatiques chlorés, les hydrocarbures cycloaliphatiques chlorés, les hydrocarbures aromatiques chlorés, les éthers aliphatiques, les éthers aromatiques, les éthers mixtes ou les mélanges de plusieurs de ces solvants.

Les acides carboxyliques pouvant être utilisés comme solvants dans le procédé de l'invention sont en particulier des monoacides ou des diacides tels que les acides aliphatiques, saturés ou insaturés, les acides aromatiques, ayant au maximum 20 atomes de carbone, dans la mesure où ils sont liquides dans les conditions opératoires. A titre d'exemples de tels acides carboxyliques, on peut citer l'acide acétique, l'acide propionique, l'acide butanoïque, l'acide valérique, l'acide méthylbutanoïque, l'acide adipique, l'acide méthylglutarique, l'acide éthylsuccinique, l'acide diméthylsuccinique, les acides penténoïques, l'acide benzoïque, l'acide phénylacétique ou les mélanges de plusieurs de ces acides. Il peut être avantageux d'utiliser des acides carboxyliques qui se forment dans la réaction d'hydroxycarbonylation du butadiène, notamment les acides penténoïques, les acides méthyl-2-buténoïques, l'acide valérique, l'acide méthylbutanoïque, l'acide adipique, l'acide méthylglutarique, l'acide éthylsuccinique, l'acide diméthylsuccinique, en particulier dans le cas du recyclage des produits de la réaction et du catalyseur.

L'acide pentène-3 oïque ou les mélanges de l'acide pentène-3 oïque avec l'acide pentène-2 oïque et/ou l'acide pentène-4 oïque et/ou d'autres sous-produits de la réaction sont plus particulièrement utilisés.

Les hydrocarbures, hydrocarbures chlorés et les éthers pouvant être utilisés sont à titre d'exemples non limitatifs, le benzène, le toluène, les xylènes, les chlorobenzènes, le dichlorométhane, le dichloroéthane, l'hexane, le cyclohexane et le diphényléther.

Dans le cadre d'une mise en oeuvre industrielle du procédé, des recyclages du catalyseur, du promoteur et du butadiène n'ayant pas réagi, peuvent conduire à l'introduction dans le milieu réactionnel de quantités plus ou moins importantes d'autres composés, notamment de sous-produits formés lors de la réaction d'hydroxycarbonylation, différents des acides carboxyliques cités précédemment. Ainsi, on peut avoir également avoir dans le mélange réactionnel des butènes et de la gamma-valérolactone. Dans le cadre de l'invention, ces composés seront considérés comme faisant partie du système solvant.

La réaction d'hydroxycarbonylation peut être conduite à une température généralement située entre 60°C et 230°C et de préférence entre 90°C et 200°C.

La pression partielle de monoxyde de carbone, mesurée à 25°C, est de 0,5 bar à 300 bar. Elle est de préférence de 2 bar à 200 bar et encore plus préférentiellement de 5 bar à 150 bar.

Comme cela a été indiqué, le procédé de l'invention peut être mis en oeuvre de manière continue ou discontinue. Selon la mise en oeuvre choisie, il y aura donc lieu d'adapter les différentes conditions opératoires définies précédemment.

Il est connu, notamment par les brevets européens n° 0 511 126 et n° 0 536 064, d'hydroxycarbonyler les acide penténoïques en acide adipique, en présence d'un catalyseur à base d'iridium et d'un promoteur iodé ou bromé. Moyennant l'adaptation des conditions opératoires à celles décrites pour cette deuxième carbonylation, il est possible de mettre en oeuvre le mélange réactionnel final, après lui avoir fait subir d'éventuels traitements, pour la préparation d'acide adipique.

Les exemples qui suivent illustrent l'invention.

### EXEMPLE 1

Dans un autoclave de 125 ml, on charge :

| | |
|---|---|
| - [IrCl(cod)]₂ | 0,42 mmol de Ir |
| - HI (solution aqueuse à 57 %) | 0,84 mmol |
| - butadiène | 2,56 g (47,4 mmol) |
| - eau | 0,88 g (48,9 mmol) |
| - dichlorométhane | 66,25 g |

L'autoclave est fermé et raccordé à un circuit d'alimentation de monoxyde de carbone. La pression est portée à 60 bar de CO et l'autoclave est chauffé à 160°C.

A cette température, la pression est ajustée à 100 bar par le monoxyde de carbone et l'essai est maintenu pendant 4 h dans ces conditions.

En fin d'essai, l'autoclave est refroidi et le mélange réactionnel est dosé par chromatographie en phase vapeur. Le mélange réactionnel final est homogène et de couleur orangé clair. Il ne comporte aucun composé goudronneux.

Les rendements (RR) obtenus correspondent au nombre de mol de composé formé pour 100 mol de butadiène engagé.

On a obtenu :

| | |
|---|---|
| - RR en acide 3-penténoïque : | 25 % |
| - RR en acide valérique : | 6 % |
| - RR en acide méthylbutanoïque : | 2 % |
| - RR en acides méthylbuténoïques : | 1 % |
| - RR en butènes : | 9 % |

### EXEMPLE 2

En opérant comme dans l'exemple 1, on réalise un essai dans l'acide 3-penténoïque comme solvant, à 140°C et sous une pression de 50 bar.

Le rapport molaire Hl/lr est de 2,5 ; la concentration initiale en eau est de 1,8 % en poids/poids.

Après 4 h de maintien en température, refroidissement du mélange réactionnel et dosage (mélange réactionnel final homogène de couleur orangée, sans présence de composés goudronneux), on a obtenu :

| | |
|---|---|
| - RR en acide 3-penténoïque : | 45 % |
| - RR en acide valérique : | 2 % |
| - RR en acide méthylbutanoïque : | 1 % |
| - RR en gamma-valérolactone : | 4 % |
| - RR en diacides à 6 atomes de C : | 19 % |

### EXEMPLE 3

En opérant comme dans l'exemple 1, on réalise un essai dans l'acide 3-penténoïque comme solvant, à 140°C et sous une pression de 200 bar.

Le rapport molaire Hl/lr est de 2,5 ; la concentration initiale en eau est de 1,8 % en poids/poids.

Après 4 h de maintien en température, refroidissement du mélange réactionnel et dosage (mélange réactionnel final homogène de couleur orangée, sans présence de composés goudronneux), on a obtenu :

| | |
|---|---|
| - RR en acide 3-penténoïque : | 40 % |
| - RR en acide valérique : | 2 % |
| - RR en acide méthylbutanoïque : | 2 % |
| - RR en gamma-valérolactone : | 5 % |
| - RR en diacides à 6 atomes de C : | 22 % |

### ESSAI COMPARATIF A

On réalise cet essai comparatif selon l'exemple 10 du brevet US 3 759 551.

On charge dans une ampoule de verre placée dans un autoclave de 125 ml :

| | |
|---|---|
| - (NH₄)₂IrCI₆ | 0,0478 g (0,108 mmol de Ir) |
| - HI (solution aqueuse à 57 %) | 2,74 g (12,2 mmol) |
| - butadiène | 5,78 g (107 mmol) |
| - eau | 0,65 g (36 mmol) |
| - acide acétique | 20 cm³ (21,8 g) |

Le rapport molaire Hl/lr est de 113 ; la concentration initiale en eau est de 9,5 % en poids/poids.

Après 17h à 165°C sous 42 barde monoxyde de carbone, le mélange réactionnel final est marron foncé avec un important dépôt de goudrons. On isole 2,1 g de ces goudrons. La partie liquide est dosée par chromatographie en phase vapeur.

On a obtenu :

| | |
|---|---|
| - RR en acides en C5 : | 0,47 % |
| dont : | |
| - RR en acide 3-penténoïque : | 0,05 % |

La quantité d'acide penténoïque formée est très faible ; les autres acides en C5 sont essentiellement l'acide valérique et l'acide méthylbutanoïque.

## Revendications

1. Procédé d'hydroxycarbonylation du butadiène, par réaction avec le monoxyde de carbone et l'eau, à une température supérieure à 30°C, sous une pression partielle en monoxyde de carbone mesurée à 25°C égale ou supérieure à 0,5 bar, en présence d'un catalyseur à base d'iridium et d'un promoteur iodé ou bromé, dans un milieu solvant, caractérisé en ce que le rapport molaire iode ou brome/iridium est inférieur ou égal à 20/1 et la concentration pondérale en eau du mélange réactionnel est inférieure ou égale à 8 %.

2. Procédé selon la revendication 1, caractérisé en ce que la concentration pondérale en eau dans le mélange réactionnel est comprise entre 0,00001 % et 5 % et de manière préférée entre 0,01 % et 2 %.

3. Procédé selon l'une des revendications 1 ou 2, caractérisé en ce que la concentration en butadiène du mélange réactionnel est maintenue à une valeur inférieure ou égale à 16 % en poids par rapport au poids total du mélange réactionnel.

4. Procédé selon l'une des revendications 1 à 3, caractérisé en ce que la concentration en butadiène dans le mélange réactionnnel est inférieure ou égale à 11 % en poids par poids et plus préférentiellement inférieure ou égale à 5,5 % en poids par poids.

5. Procédé selon l'une des revendications 1 à 4, caractérisé en ce que le catalyseur à l'iridium mis en oeuvre est choisi parmi :
- Ir métallique ; IrO₂ ; Ir₂O₃ ;
- IrCl₃ ; IrCl₃, 3H₂O ;
- IrBr₃ ; IrBr₃ ; 3H₂O ;
- les iodures d'iridium ;
- les carboxylates d'iridium, notamment l'acétate d'Ir ou le penténoate d'Ir ;
- Ir₂(CO)₄Cl₂ ; Ir₂(CO)₄I₂ ;
- Ir₂(CO)₈ ; Ir₄(CO)₁₂ ;
- Ir(CO)[P(C₆H₅)₃]₂I ;
- Ir(CO)[P(C₆H₅)₃]₂Cl ;
- Ir[P(C₆H₅)₃]₃I ;
- HIr[P(C₆H₅)₃]₃(CO) ;
- Ir(acac)(CO)₂ ;
- [IrCl(cod)]₂.

6. Procédé selon l'une des revendications 1 à 5, caractérisé en ce que la quantité de catalyseur, exprimée en moles d'iridium métal par litre de mélange réactionnel, est comprise entre 10⁻⁴ et 10⁻¹ mole/litre et de préférence est comprise entre 5.10⁻⁴ et 5.10⁻² mole/litre.

7. Procédé selon l'une des revendications 1 à 6, caractérisé en ce que le promoteur iodé ou bromé est choisi parmi l'iodure d'hydrogène, le bromure d'hydrogène et les composés organo-iodés et organo-bromés capables de générer respectivement l'iodure d'hydrogène et le bromure d'hydrogène dans les conditions réactionnelles.

8. Procédé selon l'une des revendications 1 à 7, caractérisé en ce que le rapport molaire iode ou brome/iridium est compris entre 1/1 et 10/1 et préférentiellement entre 1/1 et 5/1.

9. Procédé selon l'une des revendications 1 à 8, caractérisé en ce que le solvant, liquide dans les conditions de la réaction, est choisi parmi les acides carboxyliques, les hydrocarbures aliphatiques, les hydrocarbures cycloaliphatiques, les hydrocarbures aromatiques, les hydrocarbures aliphatiques chlorés, les hydrocarbures cycloaliphatiques chlorés, les hydrocarbures aromatiques chlorés, les éthers aliphatiques, les éthers aromatiques, les éthers mixtes ou les mélanges de plusieurs de ces solvants.

10. Procédé selon l'une des revendications 1 à 9, caractérisé en ce que la réaction d'hydroxycarbonylation est conduite à une température située entre 60°C et 230°C et de préférence entre 90°C et 200°C.

11. Procédé selon l'une des revendications 1 à 10, caractérisé en ce que la réaction d'hydroxycarbonylation est conduite sous une pression partielle de monoxyde de carbone, mesurée à 25°C, de 0,5 bar à 300 bar, de préférence de 2 bar à 200 bar et encore plus préférentiellement de 5 bar à 150 bar.

## Claims

1. Process for the hydroxycarbonylation of butadiene, by reaction with carbon monoxide and water, at a temperature above 30°C, under a partial pressure of carbon monoxide, measured at 25°C, equal to or greater than 0.5 bar, in the presence of an iridium-based catalyst and an iodine-containing or bromine-containing promoter, in a solvent medium, characterized in that the iodine or bromine/iridium molar ratio is less than or equal to 20/1 and the concentration by weight of water in the reaction mixture is less than or equal to 8 %.

2. Process according to Claim 1, characterized in that the concentration by weight of water in the reaction mixture is between 0.00001 % and 5 % and preferably between 0.01 % and 2 %.

3. Process according to either of Claims 1 and 2, characterized in that the concentration of butadiene in the reaction mixture is maintained at a value less than or equal to 16 % by weight relative to the total weight of the reaction mixture.

4. Process according to one of Claims 1 to 3, characterized in that the concentration of butadiene in the reaction mixture is less than or equal to 11 % on a weight for weight basis and, more preferably, less than or equal to 5.5 % on a weight for weight basis.

5. Process according to one of Claims 1 to 4, characterized in that the iridium catalyst used is chosen from:
- Ir metal; IrO₂; Ir₂O₃;
- IrCl₃; IrCl₃·3H₂O;
- IrBr₃; IrBr₃·3H₂O;
- iridium iodides;
- iridium carboxylates, in particular Ir acetate or Ir pentenoate;
- Ir₂(CO)₄Cl₂; Ir₂(CO)₄I₂;
- Ir₂(CO)₈; Ir₄(CO)₁₂;
- Ir(CO)[P(C₆H₅)₃]₂I;
- Ir(CO)[P(C₆H₅)₃]₂Cl;
- Ir[P(C₆H₅)₃]₃I;
- HIr[P(C₆H₅)₃]₃(CO);
- Ir(acac)(CO)₂;
- [IrCl(cod)]₂.

6. Process according to one of Claims 1 to 5, characterized in that the amount of catalyst, expressed as moles of iridium metal per litre of reaction mixture, is between 10⁻⁴ and 10⁻¹ mol/litre and is preferably between 5 x 10⁻⁴ and 5 x 10⁻² mol/litre.

7. Process according to one of Claims 1 to 6, characterized in that the iodine-containing or bromine-containing promoter is chosen from hydrogen iodide, hydrogen bromide and organoiodine and organobromine compounds capable of generating hydrogen iodide and hydrogen bromide respectively under the reaction conditions.

8. Process according to one of Claims 1 to 7, characterized in that the iodine or bromine/iridium molar ratio is between 1/1 and 10/1 and preferably between 1/1 and 5/1.

9. Process according to one of Claims 1 to 8, characterized in that the solvent, which is liquid under the reaction conditions, is chosen from carboxylic acids, aliphatic hydrocarbons, cycloaliphatic hydrocarbons, aromatic hydrocarbons, chlorinated aliphatic hydrocarbons, chlorinated cycloaliphatic hydrocarbons, chlorinated aromatic hydrocarbons, aliphatic ethers, aromatic ethers, mixed ethers or mixtures of several of these solvents.

10. Process according to one of Claims 1 to 9, characterized in that the hydroxycarbonylation reaction is carried out at a temperature between 60°C and 230°C and preferably between 90°C and 200°C.

11. Process according to one of Claims 1 to 10, characterized in that the hydroxycarbonylation reaction is carried out under a partial pressure of carbon monoxide, measured at 25°C, of 0.5 bar to 300 bar, preferably of 2 bar to 200 bar and, even more preferably, of 5 bar to 150 bar.

## Patentansprüche

1. Verfahren zur Hydroxycarbonylierung von Butadien durch Umsetzung mit Kohlenmonoxid und Wasser bei einer Temperatur über 30°C unter einem bei 25°C gemessenen Kohlenmonoxidpartialdruck von gleich oder größer 0,5 bar in Gegenwart eines Katalysators auf der Basis von Iridium und eines iodierten oder bromierten Promotors in einem Lösungsmedium, dadurch gekennzeichnet, daß das Molverhältnis Iod oder Brom /Iridium kleiner oder gleich 20/1 ist und die auf das Gewicht bezogene Konzentration an Wasser in dem Reaktionsgemisch kleiner oder gleich 8% ist.

2. Verfahren nach Anspruch 1, dadurch gekennzeichnet, daß die auf das Gewicht bezogene Konzentration an Wasser in dem Reaktionsgemisch zwischen 0,00001% und 5% und bevorzugt zwischen 0,01% und 2% liegt.

3. Verfahren nach einem der Ansprüche 1 oder 2, dadurch gekennzeichnet, daß die Konzentration an Butadien in dem Reaktionsgemisch bei einem Wert von kleiner oder gleich 16%, bezogen auf das Gesamtgewicht des Reaktionsgemisches, gehalten wird.

4. Verfahren nach einem der Ansprüche 1 bis 3, dadurch gekennzeichnet, daß die Konzentration an Butadien in dem Reaktionsgemisch kleiner oder gleich 11% (Gew./Gew.), und bevorzugter 5,5% (Gew./Gew.) ist.

5. Verfahren nach einem der Ansprüche 1 bis 4, dadurch gekennzeichnet, daß der verwendete Iridiumkatalysator ausgewählt wird aus:
- metallisches Ir; IrO₂; Ir₂O₃;
- IrCl₃; IrCl₃. 3H₂O;
- IrBr₃; IrBr₃. 3H₂O;
- Iridiumiodide;
- Iridiumcarboxylate, insbesondere Ir-acetat oder Irpentenoat;
- Ir₂(CO)₄Cl₂; Ir₂(CO)₄I₂;
- Ir₂(CO)₈; Ir₂(CO)₁₂;
- Ir(CO)[P(C₆H₅)₃]₂I;;
- Ir(CO)[P(C₆H₅)₃]₂Cl;
- Ir[P(C₆H₅)₃]₃I;;
- HIr(CO)[P(C₆H₅)₃]₃(CO);
- Ir(acac)(CO)₂;
- [IrCl(cod)]₂;

6. Verfahren nach einem der Ansprüche 1 bis 5, dadurch gekennzeichnet, daß die Menge des Katalysators, ausgedrückt als Mol Iridiummetall pro Liter Reaktionsgemisch, zwischen 10⁻⁴ und 10⁻¹ mol/l und bevorzugt zwischen 5.10⁻⁴ und 5.10⁻² mol/l liegt.

7. Verfahren nach einem der Ansprüche 1 bis 6, dadurch gekennzeichnet, daß der iodierte oder bromierte Promotor aus Iodwasserstoff, Bromwasserstoff und Organoiod- und Organobromverbindungen mit der Fähigkeit, Iodwasserstoff bzw. Bromwasserstoff unter den Reaktionsbedingungen zu erzeugen, ausgewählt wird.

8. Verfahren nach einem der Ansprüche 1 bis 7, dadurch gekennzeichnet, daß das Molverhältnis Iod oder Brom/Iridium zwischen 1/1 und 10/1 und bevorzugt zwischen 1/1 und 5/1 liegt.

9. Verfahren nach einem der Ansprüche 1 bis 8, dadurch gekennzeichnet, daß das Lösungsmittel, das unter den Reaktionsbedingungen flüssig ist, aus Carbonsäuren, aliphatischen Kohlenwasserstoffen, cycloaliphatischen Kohlenwasserstoffen, aromatischen Kohlenwasserstoffen, chlorierten aliphatischen Kohlenwasserstoffen, chlorierten cycloaliphatischen Kohlenwasserstoffen, chlorierten aromatischen Kohlenwasserstoffen, aliphatischen Ethern, aromatischen Ethern, gemischten Ethern oder Gemischen aus mehreren dieser Lösungsmittel ausgewählt wird.

10. Verfahren nach einem der Ansprüche 1 bis 9, dadurch gekennzeichnet, daß die Hydroxycarbonylierungsreaktion bei einer Temperatur zwischen 60°C und 230°C und bevorzugt zwischen 90°C und 200°C durchgeführt wird.

11. Verfahren nach einem der Ansprüche 1 bis 10, dadurch gekennzeichnet, daß die Hydroxycarbonylierungsreaktion bei einem bei 25°C gemessenen Kohlenmonoxidpartialdruck von 0,5 bar bis 300 bar, bevorzugt von 2 bar bis 200 bar und noch bevorzugter von 5 bar bis 150 bar durchgeführt wird.
